# EUROPÄISCHE PATENTSCHRIFT

(11) **EP 1 095 618 B1**
(45) Veröffentlichungstag und Bekanntmachung des Hinweises auf die Patenterteilung: **12.05.2004**
(21) Anmeldenummer: 00117494.5
(22) Anmeldetag: 14.08.2000
(51) Int. Cl.: A61B 5/22, A61B 5/0205, A61B 5/11

(54) **Verfahren und Vorrichtung zur Erstellung eines individuellen Bewegungs- und Belastungsprofils**
Method and apparatus for producing a movement and stress profile of an individual
Méthode et appareil de réalisation d'un profil des mouvements et de l'état de stress physiologique d'un individu

(30) Priorität: 29.10.1999 DE 19952164
(43) Veröffentlichungstag der Anmeldung: 02.05.2001
(73) Patentinhaber: Ganshorn, Peter, 97702 Münnerstadt (DE)
(72) Erfinder: Ganshorn, Peter, 97702 Münnerstadt (DE)
(74) Vertreter: Pöhner, Wilfried Anton, Dr.

(56) Entgegenhaltungen:
- EP-A- 0 842 635
- EP-A- 0 846 440
- WO-A-96/04848
- WO-A-99/44494
- WO-A-99/49279
- DE-A- 19 619 899
- PATENT ABSTRACTS OF JAPAN vol. 1999, no. 11, 30. September 1999 (1999-09-30) -& JP 11 166827 A (SEIKO INSTRUMENTS INC), 22. Juni 1999 (1999-06-22)

## Beschreibung

Die Erfindung bezieht sich auf ein Verfahren zur Erstellung eines individuellen Bewegungs- und Belastungsprofils an Mensch oder Tier, bei dem ein Strecken- und Höhenprofil erstellt wird.

Bewegungs- und Belastungsprofile sind unter anderem in der Arbeitsmedizin, in der Trainingslehre oder bei der Erprobung neuer Materialien für Bekleidung, wie z. B. bei neuem Schuhwerk, notwendig. Dabei wird meist die Bewegung eines Probanden in der Horizontalen erfaßt, um seine individuelle Belastung zu erfassen oder auch den Trainingsumfang zu bestimmen. Tatsächlich hängt die Belastung aber auch von der Bewegung in der Vertikalen ab sowie vom Sauerstoffgehalt der umgebenden Luft, der sich mit der absoluten Höhe über Null ändert.

Mit der Ergospirometrie können Atemvolumen, Atemfrequenz und Sauerstoffverbrauch unter Belastung bei Mensch und Tier gemessen werden. Damit wird die Reaktion des Herzkreislaufsystems auf äußere, im allgemeinen definiert vorgegebene Belastungen bestimmt. U.a. ist es bekannt, den Probanden auf einem Fahrradergometer mit einer bestimmten physikalischen Belastung zu beaufschlagen, beispielsweise muß er eine Dauerleistung von 100 W erbringen. Mit dem Ergospirometer wird dabei kontinuierlich das Atemvolumen, die Zusammensetzung der ausgeatmeten Luft sowie verschiedene kardiale Parameter, wie z. B. der Puls, erfaßt. Daraus lassen sich die tatsächlichen physiologischen Belastungen des Probanden errechnen, d.h. die tatsächliche biologische Leistung, die er erbringen muß, um am Fahrradergometer 100 Watt zu erbringen, wobei ein Mensch erfahrungsgemäß einen Wirkungsgrad von ca. 30 % aufweist, d.h. er muss im vorgenannten Beispiel ca. 330 W aufbringen. In ähnlicher Weise werden Probanden auf Laufbandergometern eine definierten Belastung unterworfen. Desweiteren sind auch tragbare Ergospirometer bekannt, die insbesondere bei Rehabilitationsmaßnahmen nach Herzkreislauferkrankungen bzw. in der Arbeits- oder Sportmedizin eingesetzt werden, bei denen der Proband ein tragbares Gerät, meist auf dem Rücken, zur Erfassung aller kardialer und pulmonaler Meßgrößen mit sich führt und die physiologische Reaktion auf verschiedene Belastungen, beispielsweise unterschiedliche Arbeitsprozesse, erfaßbar ist.

Die Kombination eines tragbaren Ergometers zur Bestimmung der physiologischen Belastung des Probanden mit einer Vorrichtung zur Erfassung der Bewegung, nicht aber der Beschleunigung, in allen drei Raumrichtungen (WO 99/49279) ist bekannt, wobei die Werte (Strecke, Geschwindigkeit) aus der periodisch wiederholten Standortbestimmung durch GPS ermittelt werden. Nicht erfaßt werden mit dieser Vorrichtung pulmonale Meßgrößen.

Als nachteilig bei den bisher bekannten Verfahren zur Erstellung ergospirometrischer Belastungsprofile ist anzusehen, daß bei den stationären Fahrrad- bzw. Laufbandergometern nur jeweils eine spezifische Belastung auf den Probanden ausgeübt werden kann. Zur Simulierung realistischer Alltagssituationen bzw. der physiologischen Reaktionen darauf beim Probanden sind diese stationären Geräte ungeeignet. Die tragbaren Ergospirometer können zwar eine kontinuierliche Messung der interessierenden Größen unter realistischen Bedingungen, beispielsweise am Arbeitsplatz oder bei einem Sportler während des Trainings, erfüllen, allerdings ist mit ihnen eine Erfassung der tatsächlichen, objektiven, äußeren Belastungen, denen der Proband ausgesetzt ist, nicht möglich, da sie jeweils immer nur die physiologische Reaktion des Probanden darauf messen. Wie es dem Fachmann bekannt ist, hängt die Reaktion des Probanden aber immer auch von individuellen Gegebenheiten ab, d.h. bei einer objektiv gleichen äußeren Belastung, werden verschiedene Probanden verschiedene physiologische Reaktionen zeigen.

Ausgehend vom Stand der Technik hat sich die Erfindung zur Aufgabe gestellt, ein Verfahren zur Erstellung eines Bewegungs- und Belastungsprofils sowie in einer Weiterbildung eines individuellen ergospirometrischen Belastungsprofils bzw. ein tragbares Ergospirometer zur Durchführung des Verfahrens zur Verfügung zu stellen, mit dem nicht nur die physiologischen Reaktionen des Probanden, sondern auch die tatsächlichen physikalischen Belastungen gemessen werden können, und die Belastung unter verschiedensten Umweltbedingungen bzw. realistischen Alltagssituationen erfaßt werden kann.

Erfindungsgemäß wird dieses Problem durch ein Verfahren gelöst, dass die Beschleunigung in allen drei Raumrichtungen durch Beschleunigungssensoren erfasst und daraus durch Weiterverarbeitung das Strecken- und Höhenprofil erstellt wird, und die Höhe über Normalnull kontinuierlich gemessen wird.

Der Kerngedanke der Erfindung besteht darin, daß zur Erstellung eines Bewegungs- und Belastungsprofils die Bewegung und Beschleunigung in allen drei Dimensionen erfaßt werden muß, um vollständige Informationen über das Bewegungsprofil des Probanden zu erhalten. Damit ist es möglich, daß beispielsweise in der Arbeitsmedizin die Bewegungsabläufe eines Arbeiters erfaßt werden, oder in der Trainingslehre der tatsächliche Umfang des Trainings eines Athleten, aber auch die Belastung bzw. die Haltbarkeit eines neuen Bekleidungsmaterials kann gemessen werden, wenn die absolute Bewegung, d. h. die tatsächliche Strecke die eine Testperson z. B. mit einem neuen Schuh zurückgelegt hat, erfaßt wird. Vorrichtungen mit denen die Beschleunigungen und/oder Bewegung erfaßt werden kann, sowie die Höhe über Null bestimmt wird, können auf vielfältige Weise ausgeführt werden, oder wie weiter unten angeführt.

Besonders von Vorteil ist das Verfahren zur Erstellung eines ergospirometrischen Belastungsprofils bei dem nicht nur die kardialen und pulmonalen Meßgrößen des Probanden mit einem an sich bekannten tragbaren Ergospirometer erfaßt werden, sondern auch die Bewegung der Probanden im Raum. Dazu werden die Höhe über Normalnull sowie die horizontale Bewegung bzw. Beschleunigung kontinuierlich erfasst. Mit diesen Meßgrößen ist ein Strecken- und Höhenprofil erstellbar, das in Verbindung mit den kardialen und pulmonalen Meßgrößen die tatsächliche Belastung des Probanden ergibt. Es ist dem Fachmann bekannt, daß beispielsweise das Bergaufgehen mit einer gewissen Geschwindigkeit eine wesentlich größere körperliche Belastung darstellt als das Gehen in der Horizontalen mit derselben Geschwindigkeit. Ebenso ist die absolute Höhe über Normalnull für die tatsächliche Belastung eine wichtige Einflussgröße, da der Sauerstoffgehalt der Luft mit zunehmender Höhe abnimmt. Die Umrechnung der Meßwerte in ein Strecken- und Höhenprofil sowie die Ableitung der tatsächlichen physikalischen Belastung des Probanden daraus ist dem Fachmann möglich. Beispielsweise muss beim Bergaufgehen zusätzliche Hubarbeit im Gegensatz zum Gehen in der Horizontalen geleistet werden. Das Gehen mit unterschiedlichen Geschwindigkeiten, die ebenfalls aus dem Streckenprofil ermittelbar sind, schlägt sich in unterschiedlichen Reaktionen des Probanden nieder.

Der Vorteil der Erfindung besteht darin, daß bei diesen Verfahren nicht nur die physiologischen Reaktionen bzw. der Sauerstoffverbruch der Probanden gemessen wird, sondern auch die tatsächliche Bewegung durch den Raum, die für die Größe der tatsächlichen physikalischen Belastung von entscheidender Bedeutung ist. Damit werden realistische, den natürlichen Bedingungen entsprechende, Meßergebnisse erzielt.

Tragbare Ergospirometer zur Durchrührung des Verfahrens sind Gegenstand von Unteransprüchen.

Zur Durchführung des Verfahrens ist ein tragbares Ergospirometer notwendig, wie es an und für sich im Stand der Technik bekannt ist, d. h., dass kardiale pulmonale und bewegungsphysikalische Messgeräten an Mensch oder Tier erfaßt werden, sowie das Ergospirometer Vorrichtungen aufweist zur Erfassung, Speicherung, Auswertung oder Übertragung dieser Daten. Die Energieversorgung übernehmen üblicherweise Akkumulatoren. Um das erfindungsgemäße Verfahren durchzuführen, sind desweiteren Vorrichtungen vorhanden, mit denen die Höhe des Probanden bzw. des Ergospirometers über Normalnull bestimmt werden kann, sowie Sensoren zur Erfassung der Bewegung und/oder Beschleunigung in allen drei Raumrichtungen. Diese Vorrichtungen bzw. Sensoren können auf unterschiedlichste Weise, wie weiter unten dargestellt, realisiert werden. Dabei ist es erfindungsgemäß unerhblich, ob die Meßwerte im Ergospirometer selbst unmittelbar weiterverarbeitet werden, oder sie lediglich gespeichert und später ausgelesen werden. Der Fachmann kann aus den Meßwerten ein Strecken- und Höheprofil erstellen bzw. die jeweils aktuelle Geschwindigkeit des Probanden bestimmen, d.h. eine Abbildung der tatsächlichen Bewegung des Probanden im Raum. Weiterhin ist ihm möglich, mit diesem Profil und den üblichen Meßwerten die tatsächliche physikalische Belastung in die biologische Belastung des Probanden umzurechnen, insbesondere bei einer Bergauf- und Bergabbewegung.

Zur Erfassung der Höhe über Normalnull wird vorgeschlagen, ein Barometer zu verwenden. Diese sind im Stand der Technik wohl bekannt und das kontinuierliche Erfassen der absoluten Höhe, bereinigt durch den sich wetterbedingt ändernden Luftdruck, ist problemlos möglich. Eine besonders einfache Ausgestaltung besteht darin, einen elektronischen Barometer, insbesondere einen piezoelektrischen, zu verwenden. Er ermöglicht, dass die elektrischen Meßsignale direkt weiterverarbeitet werden.

Zur Erfassung der Bewegung in der Horizontalen können Beschleinigungsaufnehmer oder ―sensoren auf mechanischer oder elektrischer Basis verwendet werden, die im Stand der Technik wohl bekannt sind. Auch hier ist eine kontinuierliche Erfassung der Meßgrößen bzw. deren Weiterverarbeitung in einfacher Weise möglich.

Desweiteren wird vorgeschlagen, dass das Ergospirometer mit einer Infrarotschnittstelle ausgestattet wird, um alle Meßgrößen bzw. die daraus ermittelten Daten an eine weitere Auswerte- oder Speichereinheit zu übertragen. Entsprechende Vorrichtungen sind dem Fachmann bekannt. Damit kann das tragbare Ergospirometer sowohl im mobilen Einsatz, als auch vor Ort als stationäre Einheit verwendet werden.

Weitere Einzelheiten, Merkmale und Vorteile der Erfindung lassen sich dem nachfolgenden Beschreibungsteil entnehmen, indem anhand einer Zeichnung die Erfindung näher dargelegt ist. Sie zeigt:

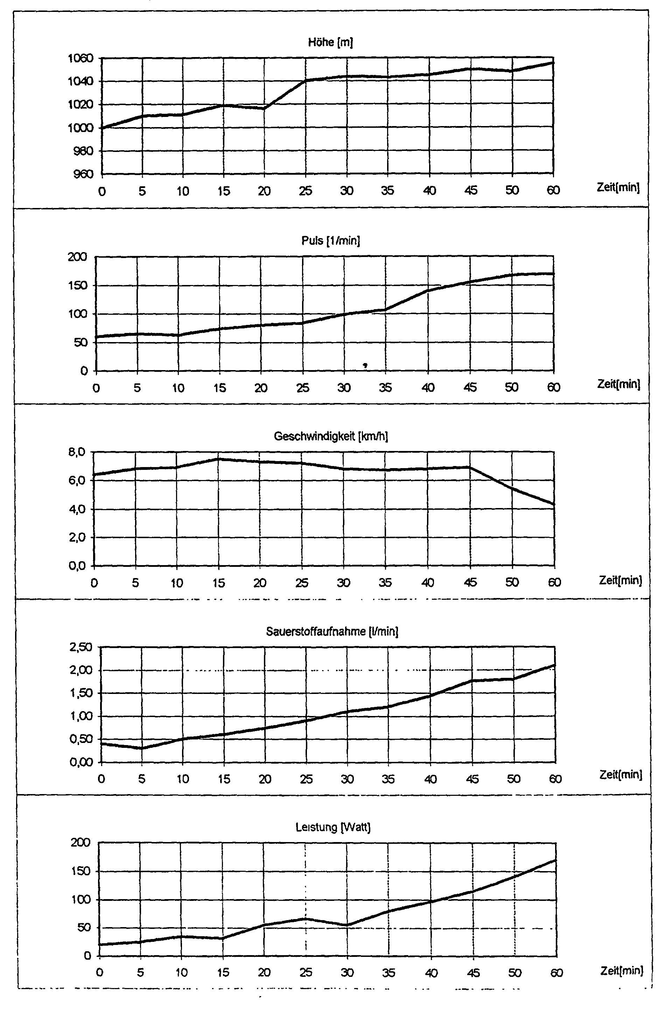

- **Figur 1**: die Belastungskurve eines Probanden, in Verbindung mit einem Streckenprofil.

Bei den in Figur 1 dargestellten Diagrammen handelt es sich um die Aufzeichnung verschiedener Meßwerte. In der ersten Zeile ist die jeweilige Höhe des Probanden über Normalnull während der Dauer der Belastungsmessung aufgezeichnet. Sie ist sowohl für den Sauerstoffgehalt der Luft ausschlaggebend, als auch dafür, daß beim Bergaufgehen mehr Leistung erbracht werden muß als in der Ebene. In der zweiten Zeile ist der Puls des Probanden aufgezeichnet, der für die biologische Belastung beziehungsweise für die physiologische Reaktion auf eine äußere Belastung besonders aussagekräftig ist. In der dritten Zeile ist die Geschwindigkeit, mit der sich der Proband in der Horizontalen fortbewegt, dargestellt. Sie ist ebenfalls für die tatsächliche physikalische Belastung bedeutend, da eine Bewegung mit größerer Geschwindigkeit eine größere Leistungsabgabe erfordert. In Zeile 4 ist die vom Probanden aufgenommene Sauerstoffmenge dargestellt. Sie wird mit Hilfe der Messung des Atemvolumens bzw. der Zusammensetzung der aus- und eingeatmeten Luft bestimmt. Mit ihr kann der Fachmann Rückschlüsse auf die biologische Leistung des Probanden ziehen. In der letzten Zeile ist die tatsächliche absolute Leistung im zeitlichen Verlauf dargestellt. Sie setzt sich zusammen aus der erbrachten biologischen Leistung sowie dem Strecken- und Höhenprofil aus dem die tatsächliche physikalische Leistung errechnet werden kann. Mit Hilfe der derart verfeinerten Meßergebnisse kann der Fachmann Rückschlüsse auf die Belastbarkeit des Organismus, also beispielsweise den Trainingszustand oder den Zustand während einer Rehabilitationsmaßnahme wesentlich genauer bestimmen.

## Patentansprüche

1. Verfahren zur Erstellung eines individuellen Bewegungs- und Belastungsprofils an Mensch oder Tier, bei dem ein Strecken- und Höhenprofil erstellt wird, **dadurch gekennzeichnet, dass**
- die Höhe über Normalnull kontinuierlich gemessen wird,
- die Beschleunigung in allen drei Raumrichtungen durch Beschleunigungssensoren erfasst und daraus durch Weiterverarbeitung das Strecken- und Höhenprofil erstellt wird.

2. Verfahren nach Anspruch 1, **dadurch gekennzeichnet, dass**
- mit einem tragbaren Ergospirometer die kardialen und pulmonalen Messgrößen erfasst werden,
- die physiologische Belastung des Probanden aus dem Strekken- und Höhenprofil errechnet und mit den kardinalen und pulmonalen Messgrößen in Bezug gesetzt wird.

3. Tragbares Ergospirometer mit Vorrichtung zur Erfassung und/oder Speicherung und/oder Auswertung und/oder Übertragung kardialer, pulmonaler und bewegungsphysikalischer Messgrössen zur Erstellung eines individuellen Bewegungs- und Belastungsprofils von Mensch oder Tier, **dadurch gekennzeichnet, dass**
- zur Erfassung der Beschleunigung in allen drei Raumrichtungen Beschleunigungssenoren vorhanden sind, und
- eine zusätzliche Vorrichtung zur Bestimmung der Höhe über Normalnull vorhanden ist.

4. Tragbares Ergospirometer nach Anspruch 3, **dadurch gekennzeichnet, dass** die Vorrichtung zur Erfassung der Höhe über Normalnull ein Barometer, insbesondere ein piezoelektrisches, ist.

5. Tragbares Ergospirometer nach einem der Ansprüche 3 und 4, **dadurch gekennzeichnet, dass** eine Infrarotschnittstelle vorhanden ist zur Übertragung aller Messgrößen an eine Auswerteeinheit.

## Claims

1. Method for preparing an individual movement and load profile on a person or animal, in which a path and altitude profile is prepared, **characterised in that**
- the height above the datum level is measured continuously
- the acceleration in all three spatial directions is recorded by means of acceleration sensors and, from this, the path and altitude profile is generated by further processing

2. Method according to claim 1, **characterised in that**
- the cardiac and pulmonary parameters are registered by means of a portable ergospirometer,
- the physiological load on the test subject is computed from the path and altitude profile, and is related to the cardiac and pulmonary parameters.

3. Portable ergospirometer comprising a device for registering and/or storing and/or evaluating and/or transmitting cardiac, pulmonary and physical movement parameters for preparing an individual movement and stress profile of a person or animal, **characterised in that**
- acceleration sensors are present for registering the acceleration in all three spatial directions, and
- an additional device is present for determining the altitude above the datum level

4. Portable ergospirometer according to claim 3, **characterised in that** the device for registering the height above the datum level is a barometer, in particular a piezoelectric barometer.

5. Portable ergospirometer according to one of claims 3 and 4, **characterised in that** an infrared interface is present for transmitting all parameters to an evaluation unit.

## Revendications

1. Procédé permettant d'établir un profil individuel de déplacement et de résistance sous effort d'un être humain ou d'un animal, ainsi qu'un profil de la distance parcourue et de l'altitude,
**caractérisé en ce que**
- le dispositif enregistre l'accélération dans les trois dimensions au moyen de capteurs, puis traite ces données pour établir un profil de la distance parcourue et de l'altitude,
- le dispositif mesure en continu la hauteur du sujet de test au-dessus du niveau de la mer.

2. Procédé selon la revendication 1, **caractérisé en ce que**
- l'on mesure les paramètres cardiaques et pulmonaires du sujet de test au moyen d'un ergospiromètre portable,
- l'on calcule la résistance physiologique du sujet de test à partir du profil de la distance parcourue et de l'altitude, compte tenu des paramètres cardiaques et pulmonaires mesurés.

3. Ergospiromètre portable avec matériel permettant d'enregistrer et/ou de mémoriser et/ou d'exploiter et/ou de transmettre les paramètres pulmonaires et physiologiques mesurés afin de pouvoir établir un profil individuel de déplacement et de résistance d'un être humain ou d'un animal, **caractérisé en ce que**
- l'appareil est équipé de capteurs enregistrant l'accélération dans les trois dimensions et
- l'appareil est doté d' un dispositif complémentaire permettant de déterminer la hauteur du sujet de test au-dessus du niveau de la mer.

4. Procédé selon la revendication 3, **caractérisé en ce que** le dispositif mesurant la hauteur du sujet de test au-dessus du niveau de la mer est un baromètre, notamment un baromètre piézoélectrique.

5. Procédé selon l'une des revendications 3 et 4, **caractérisé en ce que** le dispositif est équipé d'une interface infrarouge permettant de transmettre toutes les grandeurs mesurées à un module d'exploitation.
